# EUROPÄISCHE PATENTSCHRIFT

(11) **EP 0 973 460 B1**
(45) Veröffentlichungstag und Bekanntmachung des Hinweises auf die Patenterteilung: **02.10.2002**
(21) Anmeldenummer: 97953911.1
(22) Anmeldetag: 23.12.1997
(51) Int. Cl.: A61C 17/22, A46B 15/00, A61K 7/16, B08B 3/10

(54) **REINIGUNGSSYSTEM UND VERFAHREN ZUM REINIGEN EINER OBERFLÄCHE**
CLEANING SYSTEM AND SURFACE CLEANING METHOD
SYSTEME DE NETTOYAGE ET PROCEDE POUR LE NETTOYAGE D'UNE SURFACE

(30) Priorität: 23.12.1996 DE 19654108
(43) Veröffentlichungstag der Anmeldung: 26.01.2000
(73) Patentinhaber: Massholder, Karl F., Dr., 69250 Schönau (DE)
(72) Erfinder: MASSHOLDER, Karl, D-69250 Schönau (DE); MANNSCHOTT, Peter, D-69250 Schönau (DE)
(74) Vertreter: Isenbruck, Günter, Dr.
(86) Internationale Anmeldenummer: EP9707254
(87) Internationale Veröffentlichungsnummer: WO98027891

(56) Entgegenhaltungen:
- EP-A- 0 743 029
- WO-A-92/06671
- US-A- 4 983 379

## Beschreibung

Die Erfindung betrifft ein Reinigungssystem sowie ein Verfahren zum Reinigen einer Oberfläche.

In vielen Bereichen des täglichen Lebens spielt die Hygiene eine wichtige Rolle. Eine gute Hygiene kann dazu beitragen, daß eine Reihe von Krankheiten verhindert wird, indem Keime, die sich an Oberflächen ansiedeln, entfernt oder vernichtet werden. Ein weiterer Aspekt ist Sauberkeit. Beim Reinigen von Oberflächen werden optisch störende Verunreinigungen entfernt, so daß sich ein angenehmeres Äußeres ergibt. Gelegentlich ist es auch notwendig, Ablagerungen oder angelagerte Stoffe zu entfernen, die sich aufgrund einer Umweltverschmutzung ergeben haben.

Bisherige Reinigungsverfahren beruhen in der Regel darauf, daß man die Oberfläche mechanisch abreinigt, d.h. ein Reinigungswerkzeug über die Oberfläche führt und dabei Verunreinigungen mechanisch ablöst und abträgt. Zur Vernichtung oder zur Abtötung von Keimen und Bakterien werden vielfach chemische Mittel eingesetzt, die dann ihrerseits wieder entfernt werden müssen und teilweise ein aggressives Verhalten gegenüber der zu reinigenden Oberfläche haben. In vielen Fällen muß man daher bei der Wahl der Reinigungs- oder Putzmittel sehr sorgfältig vorgehen und diese auf die zu reinigende Oberfläche entsprechend abstimmen. Insbesondere bei der Bekämpfung von Keimen und Bakterien ist aber zu beobachten, daß bestimmte Keime eine immer stärkere Resistenz gegen bestimmte Mittel entwickeln, die zu ihrer Bekämpfung eingesetzt werden. Auch nach erfolgter Reinigung ist dann die gewünschte Keimarmut oder sogar Keimfreiheit nicht erreicht, auch wenn dies gar nicht unmittelbar bemerkt wird.

Aus EP-A- 0 743 029 ist ein Reinigungssystem bekannt, das eine, gegenüber einer mechanischen Reinigung hinausgehende Reinigung ermöglicht, die auf der Freisetzung von hyperaktivem Singulett- Sauerstoff aus einem in einer Dentalflüssigkeit oder Paste enthaltenen Fotosensibilisator unter Einwirkung von Laserlicht basiert.

Aus US-A- 4,983,379 ist die Verwendung von Titandioxid als stabilisierender glättender Zusatz zu einer Paste zur Behandlung von Zahnfleischerkrankungen bekannt.

Die WO-A- 92/06671 beschreibt ein Verfahren und eine Vorrichtung zur Feststellung von Zahnbelag und zur Zahnreinigung, das auf dem Zusammenwirken von sichtbarem Licht bestimmter Wellenlänge und einem fluoreszierenden Additiv in der beim Diagnose- und Reinigungsvorgang aufzubringenden Zahnpaste beruht.

Der Erfindung liegt die Aufgabe zugrunde, ein Reinigungssystem anzugeben, das es ermöglicht, die Reinigung von Oberflächen zu vereinfachen, die Zuverlässigkeit der Reinigungswirkung zu erhöhen und Nebenwirkungen weitgehend auszuschließen. Weiterhin ist es Aufgabe der Erfindung, ein verbessertes Verfahren zum Reinigen einer Oberfläche anzugeben. wobei Verfahren zum Reinigen der Zähne ausgeschlossen sind.

Die Lösung geht aus von einem Reinigungssystem zum Reinigen einer Oberfläche, das aufweist:
- ein Reinigungswerkzeug (2) mit einem Arbeitsbereich (A) und einer UV-Lichtquelle (7), deren UV-Strahlung im Arbeitsbereich (A) aus dem Werkzeug (2) austritt und
- ein Reinigungsmittel, das ein fotoaktivierbares Halbleitermaterial (3) enthält.

Das erfindungsgemäße Reinigungssystem ist dann gekennzeichnet durch eine Lichtleiteinrichtung (9, 14), über die UV-Strahlung im Arbeitsbereich (A) unmittelbar in das fotoaktivierbare Halbleitermaterial (3) eingekoppelt wird.

Das erfindungsgemäße Reinigungssystem ist somit derart ausgestaltet, daß UV-Licht aus einer UV-Lichtquelle durch eine Lichtleiteinrichtung im Arbeitsbereich des Reinigungswerkzeugs unmittelbar in das fotoaktivierbare Halbleitermaterial eingekoppelt wird. Auf diese Weise wird eine erhöhte Effektivität der Reinigungswirkung sowie eine weitgehende Vermeidung von Nebenwirkungen erreicht.

Eine Gefährdung von Menschen durch UV-Strahlung ist ausgeschlossen. Die UV-Strahlung wird unmittelbar zur zu reinigenden Oberfläche geleitet und tritt erst dort aus dem Lichtleiter aus. Weiterhin wird die UV-Strahlung auf kleine Flächenbereiche der Oberfläche konzentriert, so daß die zur Erzeugung der UV-Strahlung notwendige Energie gut ausgenutzt wird.

Die Vorgehensweise bei der Anwendung des erfindungsgemäßen Reinigungssystems zum Reinigen einer Oberfläche ist überaus einfach:

Es reicht zunächst einmal aus, einen fotoaktivierbaren Halbleiter, beispielsweise in Pulverform oder in Form einer Suspension oder Flüssigkeit auf die zu reinigende Oberfläche aufzubringen. Man muß dann nur noch dafür sorgen, daß UV-Strahlung über einen Lichtleiter unmittelbar an die Oberfläche gelangt. Obwohl die Vorgänge noch nicht abschließend geklärt sind, nimmt man an, daß die UV-Strahlung dann den Halbleiter aktiviert, d.h. eine Änderung der Elelttronenkorfiguration der Halbleitermoleküle bewirkt. Fotoaktivierung bedeutet, daß durch die Lichtabsorption im Halbleiter, z.B. n-TiO₂, Elektronen vom Valenzband in das Leitungsband gehoben werden. Hierdurch entsteht ein Redoxpotential, das über die Bildung radikalischer Spezies bzw. Mechanismen zur Abtötung von Mikroorganismen führt. Da diese Prozesse unspezifisch sind, kommt es nebenbei auch zu oxidativen Abbaureaktionen. Da sich der Halbleiter nicht verändert spricht man von einem Katalysator. Man kann also dieses Verfahren mit ausgezeichneten Ergebnissen für die Desinfektion verwenden. Man kann es aber auch verwenden, um oxidierbare Stoffe, beispielsweise Kohlenwasserstoffe, zu oxidieren. So lassen sich beispielsweise Ölflecken dadurch entfernen, daß man den fotoaktivierbaren Halbleiter in Pulverform aufstreut und dann UV-Licht entweder von der Sonne oder einer UV-Lichtquelle darauf wirken läßt. Das Öl wird oxidiert und zersetzt sich dann weitgehend in Kohlendioxid und Wasser.

Vorzugsweise verwendet man hierbei als Lichtleiter für die UV-Strahlung ein mechanisches Reinigungsinstrument. Neben der Oxidation der Verunreinigungen bzw. Keime hat man dann gleichzeitig ein Werkzeug zur Verfügung, mit dem man diese oxidierten Verunreinigungen oder Keime mechanisch abtragen kann. Man kombiniert hier also die Vorteile einer herkömmlichen Reinigung mit den Vorteilen der "oxidativen" Reinigung. Hierdurch lassen sich die Reinigungszeiten kurz halten.

Bevorzugterweise verwendet man als Reinigungsinstrument eine Bürste, bei der mindestens einige Borsten als Lichtleitfasern ausgebildet sind. Bei dieser Ausgestaltung dienen die Borsten, wie bei herkömmlichen Bürsten auch, als mechanische Reinigungswerkzeuge. Zusätzlich dienen die Borsten oder zumindest einige Borsten aber auch dazu, das UV-Licht bis an die Oberfläche zu leiten, wo es zusammen mit dem fotoaktivierbaren Halbleiter zu einer oxidativen Reinigung verwendet wird. Die Kombination des mechanischen Reinigens mit der Oxidation der zu bekämpfenden Verschmutzungen steigert die Wirkung einer herkömmlichen Bürste insbesondere im Hinblick auf die Bekämpfung von Keimen.

Diese Ausgestaltung hat darüber hinaus noch den Vorteil, daß die Borsten den Halbleiter beim Durchführen der Reinigung immer wieder von einem Punkt zum anderen verschieben, so daß mit einer hohen Wahrscheinlichkeit sichergestellt wird, daß sämtliche Keime bzw. sämtliche Verschmutzungen auf der Oberfläche erfaßt und oxidiert werden können.

Mit besonderem Vorteil ist die Bürste als Zahnbürste ausgebildet, wobei die UV-Lichtquelle im Handgriff der Zahnbürste angeordnet ist. Dies ergibt eine sehr kleine Baugröße, so daß die Zahnbürste nicht über die Baugröße einer bisherigen Zahnbürste hinausgeht. Die elektrischen Bauteile, die zur Erzeugung der UV-Strahlung in der Regel notwendig sind, und wasserführende Teile an der Zahnbürste sind sicher getrennt. Man kann das Innere des Handgriffs recht problemlos wasserdicht ausgestalten, wenn man die UV-Strahlung mit einem Lichtleiter in das Borstenfeld führt. Die Putzgewohnheiten müssen gegenüber einer herkömmlichen Zahnbürste praktisch nicht geändert werden. Bakterien auf Zähnen, Zahnfleisch und vor allem auch in Zahn-Zwischenräumen werden vernichtet, auch wenn die Borsten nicht unmittelbar in die Zahnzwischenräume vordringen. Als positiver Nebeneffekt hat sich herausgestellt, daß die Zähne ohne chemische Hilfsmittel weiß werden. Offensichtlich werden also neben den Keimen auch andere organische Verunreinigungen, die unter anderem die Zähne verfärben, oxidativ entfernt. Man kann also beispielsweise speziell für Raucher ein geeignetes System mit "Raucherzahnpasta" und Zahnbürste zusammenstellen. Durch die abrasive Wirkung der Putzkörper aus Halbleitermaterial erzielt man auch einen mechanischen Abtrag der Verschmutzungen.

Vorzugsweise wird das Halbleitermaterial in Form von Putzkörpern oder zusammen mit Putzkörpern verwendet. Es unterstützt daher nicht nur die Oxidation der Verunreinigungen oder der zu entfernenden Verschmutzung, sondern trägt auch mit zur mechanischen Reinigung bei.

Vorzugsweise wird das Halbleitermaterial in pastöser Form oder als Bestandteil einer Paste verwendet. Das Halbleitermaterial haftet dann besser an dem Untergrund. d.h. der zu reinigenden Oberfläche. Dies ist immer dann von Vorteil, wenn die Oberfläche nicht waagrecht liegt und die Reinigung in Schwerkraftrichtung von oben erfolgt, also beispielsweise bei Wänden, die senkrecht stehen oder eine gewisse Neigung aufweisen oder bei Decken oder anderen Flächen, die überhängen.

In einer alternativen Ausgestaltung kann man das Halbleitermaterial als Schwimmkörper ausbilden oder es an einen Schwimmkörper binden. Damit lassen sich nun auch Oberflächen reinigen, die bislang einer mechanischen Reinigung schwer zugänglich waren, nämlich die Oberflächen von Flüssigkeiten. beispielsweise von Seen, Flüssen oder Meeren. Vielfach treten Ölflecke auf Wasseroberflächen auf, sei es durch defekte Boote oder Schiffe, sei es durch das bewußte oder fahrlässige Entleeren von Ölresten in das Wasser. Die mechanische Entfernung dieser Ölflecken ist nur sehr schwierig und mit hohem Aufwand möglich. Wenn man nun das Halbleitermaterial schwimmfähig ausgestaltet, dann kann die katalytische Wirkung des Halbleitermaterials, die unter der Wirkung des im Sonnenlicht enthaltenen UV-Lichts zustande kommt, dazu verwendet werden, derartige Verschmutzungen oxidativ abzubauen und damit zu entfernen.

Hierbei ist besonders bevorzugt, daß als Schwimmkörper ein mineralisches Material, ein organisches Material oder eine gallertartige Flüssigkeit mit einem spezifischen Gewicht kleiner als 1 g/cm³ verwendet wird. Als mineralisches Material kommt beispielsweise Blähton, Perlitt, Gasbeton, Lava, Bims oder Kieselgur in Betracht. Als organisches Material kann man pflanzliche Produkte verwenden, beispielsweise Popcorn. Als Flüssigkeit kann man Gallerten verwenden, die das Halbleitermaterial einbinden, das UV-Licht durchlassen und trotzdem schwimmen. Derartige Schwimmkörper haben nämlich den Vorteil, daß sie nicht zu einer zusätzlichen Umweltverschmutzung führen, sondern nach dem Abbau der Verschmutzungen entweder biologisch abgebaut werden können oder absinken und sedimentieren.

Vorzugsweise weist die UV-Strahlung eine Wellenlänge im Bereich von 280 bis 400 nm, insbesondere im Bereich von 320 - 380 nm auf. Diese UV-Strahlung ist im Sonnenlicht enthalten. Sie ist für den Menschen weitgehend ungefährlich. Sie wird sogar für kosmetische und medizinische Zwecke eingesetzt. Man kann daher diese UV-Strahlung auch dann verwenden, wenn Oberflächen im oder am menschlichen Körper gereinigt werden sollen, beispielsweise die Oberflächen der Zähne im Mund.

Als fotoaktivierbarer Halbleiter wird vorzugsweise Titandioxid oder Siliziumcarbid verwendet. Beide Halbleiter sind relativ preisgünstig und in großen Mengen verfügbar.

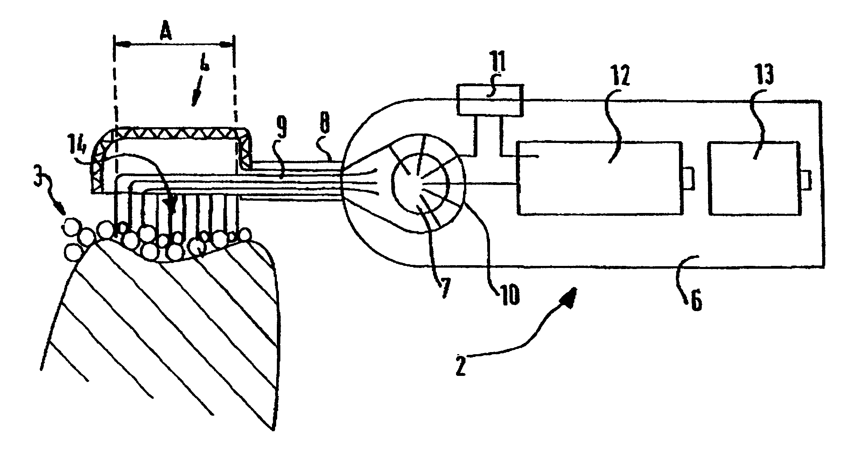

Die Erfindung wird im folgenden anhand eines bevorzugten Ausführungsbeispiels in Verbindung mit der Zeichnung beschrieben. Hierin zeigt die
- einzige Figur: eine schematische Darstellung eines Reinigungssystems mit einer Zähnbürste.

Ein Reinigungssystem 1 weist eine Zahnbürste 2 und ein fotoaktivierbares Halbleitermaterial 3 auf. Dargestellt ist die Zahnbürste 2 mit ihrem Kopf 4 über einem Zahn 5, dessen Oberflächen gereinigt werden sollen.

Die Zahnbürste 2 weist einen Griff 6 auf, in dem eine UV-Lichtquelle 7 angeordnet ist. Die UV-Lichtquelle erzeugt UV-Strahlung mit einer Wellenlänge im Bereich von 320 bis 400 nm. Diese UV-Strahlung wird über eine im Stiel 8 angeordnete Lichtleiteinrichtung 9 zum Bürstenkopf 4 geleitet. Zusätzlich ist ein Reflektor 10 vorgesehen, der die UV-Strahlung in den Eingang der Lichtleiteinrichtung 9 richtet.

Die UV-Lichtquelle kann über einen Schalter 11 aktiviert werden. Der Schalter 11 ist in einem Strompfad zwischen der UV-Lichtquelle 7 und Batterien 12, 13 angeordnet, die sich ebenfalls im Handgriff 6 der Zahnbürste befinden.

Die Lichtleiteinrichtung 9 weist eine Reihe von lichtleitenden Fasern auf, die gleichzeitig als Borstenmaterial verwendet werden können. Ein geeigneter Kunststoff hierfür ist beispielsweise Polyacrylamid. Dieser Kunststoff ist einerseits in der Lage, UV-Licht im angegebenen Wellenlängebereich zu leiten. Andererseits ist er aber auch stabil genug, um als Zahnbürstenborste funktionieren zu können.

Im Zahnbürstenkopf sind also eine oder mehrere Borstenarten vorhanden: Zumindest einige Borsten 14 bilden Teil der Lichtleiteinrichtung 9. Es kann daneben auch andere Borsten geben, die nicht als Lichtleiter dienen, sondern lediglich die Funktion der mechanischen Abreinigung haben.

Auf dem Zahn 5 sind schematisch Putzkörperchen dargestellt, die zumindest teilweise aus einem fotoaktivierbaren Halbleitermaterial 3, beispielsweise Titandioxid oder Siliziumcarbid bestehen. Man kann entweder alle Putzkörperchen einheitlich aus dem Halbleitermaterial bilden oder man kann das Halbleitermaterial neben anderen Putzkörperchen vorsehen. Natürlich ist es auch möglich, das Halbleitermaterial nicht als Putzkörperchen auszubilden, sondern sie neben den Putzkörperchen in der pastösen Masse einer Zahncreme unterzubringen.

Die Zähne können nun auf herkömmliche Art und Weise geputzt werden. Beim Putzen wird die UV-Lichtquelle 7 durch Betätigen des Schalters 11 eingeschaltet. In einem mit A gekennzeichneten Arbeitsbereich trifft dann die UV-Strahlung auf das Halbleitermaterial 3, das dadurch aktiviert wird. Es entstehen freie Valenzen, die zur Folge haben, daß Verunreinigungen, Keime oder Bakterien, die sich auf der Zahnoberfläche befinden, oxidiert und damit inaktiviert werden. Dieses Vorgehen hat den Vorteil, daß die Zähne auch an solchen Stellen gereinigt werden, wo die Borsten nicht direkt hinkommen, beispielsweise in Zahnzwischenräumen. Das Halbieitermatetial 3 wird aber in der Regel mit der im Mund beim Zähneputzen vorhandenen oder sich entwickelnden Flüssigkeit auch in solche Räume hineingespült werden. Solange die UV-Strahlung dorthin gelangt, erfolgt dort eine Reinigung durch Odxidation.

Beim Putzen wird die Schicht, die die Zahncreme, in der das Halbleitermaterial 3 enthalten ist, zumindest lokal sehr stark verdünnt, so daß das mit einer ausgesprochen hohen statistischen Wahrscheinlichkeit immer aktiviertes Halbleitermaterial 3 an die Oberfläche des Zahns 5 gelangt und dort die Reinigungswirkung durch Oxidation entfalten kann.

Natürlich lassen sich auf die gleiche Weise auch andere Flächen reinigen, wenn man entsprechendes Putzmittel, das das fotoaktivierbare Halbleitermaterial enthält und ein entsprechend ausgebildete Bürste enthält. Anstelle einer Bürste kann man auch einen Schaber verwenden, wenn man dafür sorgt, daß an seiner Schabekante oder zumindest in der unmittelbaren Umgebung das UV-Licht austreten und auf die zu reinigende Oberfläche treffen kann.

## Patentansprüche

1. Reinigungssystem zum Reinigen einer Oberfläche, das aufweist:
- ein Reinigungswerkzeug (2) mit einem Arbeitsbereich (A) und einer Strahlungsquelle (7), sowie mit einer Lichtleiteinrichtung (9,14), über die Strahlung von der Strahlungsquelle (7) zur im Arbeitsbereich (A) gelegenen Austrittsstelle aus dem Reinigungswerkzeug (2) geleitet wird und
- ein Reinigungsmittel,
**dadurch gekennzeichnet, daß** die Strahlungsquelle (7) eine UV-Strahlungsquelle ist, daß das Reinigungsmittel ein fotoaktivierbares Halbleitermaterial (3) enthält und daß die UV-Strahlung an der Austrittsstelle aus dem Reinigungswerkzeug (2) unmittelbar in das fotoaktivierbare Halbleitermaterial (3) eingekoppelt wird.

2. Reinigungssystem nach Anspruch 1, **dadurch gekennzeichnet, daß** man als Lichtleiteinrichtung (9, 14) für die UV-Strahlung ein mechanisches Reinigungswerkzeug (2) verwendet.

3. Reinigungssystem nach Anspruch 2, **dadurch gekennzeichnet, daß** man als Reinigungswerkzeug (2) eine Bürste verwendet, bei der mindestens einige Borsten (14) als Lichtleitfasern ausgebildet sind.

4. Reinigungssystem nach Anspruch 3, **dadurch gekennzeichnet, daß** die Bürste (2) als Zahnbürste ausgebildet ist, wobei die UV-Lichtquelle (7) im Handgriff (6) der Zahnbürste angeordnet ist.

5. Reinigungssystem nach einem der Ansprüche 1 bis 4, **dadurch gekennzeichnet, daß** das Halbleitermaterial (3) in Form von Putzkörpern oder zusammen mit Putzkörpern verwendet wird.

6. Reinigungssystem nach einem der Ansprüche 1 bis 5, **dadurch gekennzeichnet, daß** das Halbleitermaterial (3) in pastöser Form oder als Bestandteil einer Paste verwendet wird.

7. Reinigungssystem nach einem der Ansprüche 1 bis 6, **dadurch gekennzeichnet, daß** man ein an Schwimmkörper gebundenes oder als Schwimmkörper ausgebildetes Halbleitermaterial (3) verwendet.

8. Reinigungssystem nach Anspruch 7, **dadurch gekennzeichnet**, da als Schwimmkörper ein mineralisches Material, ein organisches Material oder eine gallertartige Flüssigkeit mit einem spezifischen Gewicht kleiner als 1 g/cm³ verwendet wird.

9. Reinigungssystem nach einem der Ansprüche 1 bis 8, **dadurch gekennzeichnet, daß** als fotoaktivierbarer Halbleitermaterial (3) Titandioxid oder Siliziumcarbid verwendet wird.

10. Reinigungssystem nach einem der Ansprüche 1 bis 9, **dadurch gekennzeichnet, daß** die Strahlung der UV-Lichtquelle (7) eine Wellenlänge im Bereich von 280 nm - 400 nm, insbesondere im Bereich von 320 - 380 nm aufweist.

11. Verwendung des Reinigungssystems nach Anspruch 1 bis 10 zum Reinigen einer Oberfläche mit Ausnahme der Verwendung dieses Systems zu therapeutischen Zwecken.

## Claims

1. Cleaning system for cleaning a surface, the said cleaning system having:
- a cleaning tool (2) with a working region (A) and with a radiation source (7), and also with a light guide device (9, 14), via which radiation is guided from the radiation source (7) to the exit point from the cleaning tool (2), the said exit point being located in the working region (A), and
- a cleaning agent,
**characterized in that** the radiation source (7) is a UV-radiation source, **in that** the cleaning agent contains a photoactivatable semiconductor material (3), and **in that** the UV-radiation is fed directly into the photoactivatable semiconductor material (3) at the exit point from the cleaning tool (2).

2. Cleaning system according to Claim 1, **characterized in that** a mechanical cleaning tool (2) is used as a light guide device (9, 14) for the UV-radiation.

3. Cleaning system according to Claim 2, **characterized in that** a brush, in which at least some bristles (14) are designed as optical fibres, is used as a cleaning tool (2).

4. Cleaning system according to Claim 3, **characterized in that** the brush (2) is designed as a toothbrush, the UV-light source (7) being arranged in the handle (6) of the toothbrush.

5. Cleaning system according to one of Claims 1 to 4, **characterized in that** the semiconductor material (3) is used in the form of cleaning bodies or together with cleaning bodies.

6. Cleaning system according to one of Claims 1 to 5, **characterized in that** the semiconductor material (3) is used in pasty form or as an ingredient of a paste.

7. Cleaning system according to one of Claims 1 to 6, **characterized in that** a semiconductor material (3) bound to floating bodies or designed as floating bodies is used.

8. Cleaning system according to Claim 7, **characterized in that** a mineral material, an organic material or a jelly-like liquid with a specific gravity lower than 1 g/cm³ is used as a floating body.

9. Cleaning system according to one of Claims 1 to 8, **characterized in that** titanium dioxide or silicon carbide is used as a photoactivatable semiconductor material (3).

10. Cleaning system according to one of Claims 1 to 9, **characterized in that** the radiation of the UV-light source (7) has a wavelength in the range of 280 nm - 400 nm, in particular in the range of 320 - 380 nm.

11. Use of the cleaning system according to Claims 1 to 10 for cleaning a surface, with the exception of the use of this system for therapeutic purposes.

## Revendications

1. Système de nettoyage pour le nettoyage d'une surface, comprenant :
- un outil de nettoyage (2) avec un domaine de travail (A) et une source de rayonnement (7), ainsi qu'un dispositif guide de lumière (9, 14) par lequel le rayonnement est guidé hors de l'outil de nettoyage (2) depuis la source de rayonnement (7) vers une zone de sortie s'étendant dans le domaine de travail, et
- un agent de nettoyage,
**caractérisé en ce que** la source de rayonnement (7) est une source de rayonnement UV, **en ce que** l'agent de nettoyage contient un matériau semiconducteur photoactivable (3) et **en ce que** le rayonnement UV à la zone de sortie hors de l'outil de nettoyage (2) est appliqué directement au matériau semiconducteur photoactivable (3).

2. Système de nettoyage selon la revendication 1, **caractérisé en ce que** l'on utilise un outil de nettoyage mécanique (2) comme dispositif guide de lumière (9, 14) pour le rayonnement UV.

3. Système de nettoyage selon la revendication 2, **caractérisé en ce que** l'on utilise comme outil de nettoyage (2) une brosse où au moins certains des poils (14) sont conformés en fibres guides de lumière.

4. Système de nettoyage selon la revendication 3, **caractérisé en ce que** la brosse (2) est conformée en brosse à dents, où la source de lumière UV (7) est disposée dans la poignée (6) de la brosse à dents.

5. Système de nettoyage selon l'une des revendications 1 à 4, **caractérisé en ce que** le matériau semiconducteur (3) est utilisé sous forme de substance de nettoyage ou avec une substance de nettoyage.

6. Système de nettoyage selon l'une des revendications 1 à 5, **caractérisé en ce que** le matériau semiconducteur (3) est utilisé sous forme pâteuse ou comme composante d'une pâte.

7. Système de nettoyage selon l'une des revendications 1 à 6, **caractérisé en ce que** l'on utilise un matériau semiconducteur (3) relié à un corps flottant ou conformé en corps flottant.

8. Système de nettoyage selon la revendication 7, **caractérisé en ce que** l'on utilise comme corps flottant une matière minérale, une matière organique ou un liquide gélatineux de masse spécifique inférieure à 1 g/cm³.

9. Système de nettoyage selon l'une des revendications 1 à 8, **caractérisé en ce que** l'on utilise comme matériau semiconducteur photoactivable (3) le dioxyde de titane ou le carbure de silicium.

10. Système de nettoyage selon l'une des revendications 1 à 9, **caractérisé en ce que** le rayonnement de la source de lumière UV (7) présente une longueur d'onde comprise dans la plage 280 nm - 400 nm, en particulier dans la plage 320 - 380 nm.

11. Utilisation du système de nettoyage selon les revendications 1 à 10 pour le nettoyage d'une surface, à l'exception de l'utilisation de ce système à des fins thérapeutiques.
